(19) Europäisches Patentamt / European Patent Office / Office européen des brevets

(11) **EP 4 458 304 A1**

(12) **EUROPEAN PATENT APPLICATION**

(43) Date of publication:
**06.11.2024 Bulletin 2024/45**

(21) Application number: **23171634.1**

(22) Date of filing: **04.05.2023**

(51) International Patent Classification (IPC):
**A61B 34/10** $^{(2016.01)}$    **G06T 7/10** $^{(2017.01)}$

(52) Cooperative Patent Classification (CPC):
**A61B 34/10;** A61B 2034/104; A61B 2034/105

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Siemens Healthineers AG**
**91301 Forchheim (DE)**

(72) Inventors:
• **Sudarsky, Sandra**
  **Bedminster, 07921 (US)**
• **Petkov, Kaloian**
  **Lawrenceville, 08648 (US)**

(74) Representative: **Siemens Healthineers**
**Patent Attorneys**
**Postfach 22 16 34**
**80506 München (DE)**

(54) **COMPUTER IMPLEMENTED METHOD FOR PLANNING A LIVER RESECTION**

(57)    Computer implemented method for planning a liver resection, comprising the steps of:
- Receiving a three-dimensional medical image dataset (1) comprising a depiction of a liver (2) of a patient, the depicted liver (2) comprising a tumor (3),
- segmenting multiple functional segments (4-12) of the liver (2) in the medical image dataset (1),
- segmenting of the tumor (3) in the medical image dataset (1),
- selecting at least one of the functional segments (4-12) that comprises the tumor (3) or into which the tumor (3) extends, based on the segmentation of the tumor (3) and the segmentation of the functional segments (4-12),
- selecting for the respective selected functional segment (12), whether a complete removal or a partial removal of this selected functional segment (12) is to be performed in a proposed liver resection plan (13), wherein the selection is based on the segmented tumor (3), and
- providing the proposed liver resection plan (13).

FIG 4

EP 4 458 304 A1

**Description**

The invention concerns a computer implemented method for planning a liver resection, a data processing system, a computer program, and a computer-readable medium.

Liver cancer is one of the most common life-threatening illnesses in the world. Recent advances in imaging technologies have resulted in an earlier detection, better treatments, and improved outcomes for liver disease patients. A surgical removal of a, especially malignant, tumor, typically called liver resection, often constitutes the most effective option to treat liver cancer.

The primary challenge of liver resection planning is to find a compromise between the competing objectives of removing the tumor completely given some safety margins, of preserving as much healthy liver tissue as possible, of preserving major vascular structures and of defining resections that are surgically possible, especially at a given skill level of a given clinician performing the surgery.

Virtual liver resections systems are, e.g., described in Mise, Y. et al., Virtual liver resection: computer-assisted operation planning using a three-dimensional liver representation, J Hepatobiliary Pancreat Sci. 20, 157-164 (2013) and in Christ B. et al., Computational Modeling in Liver Surgery, Front Physiol., Vol. 8 (2017). The tools described in these publications support surgical planning by visualizing the affected vasculature and safety margins around tumors and by computing volumetric measurements of the remanent volume. While these tools can provide real-time visual feedback during the planning process, clinicians are still required to manually specify the resection proposals.

The invention is therefore based on the problem of further improving of the planning of a liver resection.

The problems solved by a computer implemented method for planning a liver resection, comprising the steps of

- receiving a three-dimensional medical image dataset comprising a depiction of a liver of a patient, the depicted liver comprising a tumor,
- segmenting multiple functional segments of the liver in the medical image dataset,
- segmenting of the tumor in the medical image dataset,
- selecting at least one of the functional segments that comprises the tumor or into which the tumor extends, based on the segmentation of the tumor and the segmentation of the functional segments,
- selecting for the respective selected functional segment, whether a complete removal or a partial removal of this selected functional segment is to be performed in a proposed liver resection plan, wherein the selection is based on the segmented tumor, and
- providing the proposed liver resection plan.

The method allows to automatically define an advantageous proposed liver resection plan based on a functional subdivision of the liver. The automatic generation of a proposed liver resection plan can be used to support a clinician during the preparation of a medical procedure. Additionally or alternatively, the method can also be used to aid the decision making, e.g., when comparing a liver resection to other potential treatment options like, e.g., ablation or radioembolization, e.g., when discussing treatment options during a cancer board. Since the method allows for a fast determination of a proposed liver resection plan, the decision making can be based on concrete planning and/or available skill levels that influence the planning.

Due to the automatic selection of affected functional segments and the automatic selection between a complete removal and a partial removal of the respective selected functional segment, the time required for planning a liver resection can be strongly reduced. Since the automatic planning is preferably performed even before central decisions concerning the resection are made, e.g., which blood vessels to preserve, then proposed method is also helpful to provide ideas for operational approaches that the clinician might have missed in a manual planning of the resection.

The segmentation of the tumor is preferably performed automatically and can, e.g., be based on image features caused by a high concentration of a contrast agent or a molecular imaging agent within the tumor and/or on other properties that differ between the tumor and healthy liver tissue, e.g., a water content, a density, etc.

The segmentation of the functional segments of the liver can be performed automatically, especially without any user input. The segmentation is preferably performed according to the Couinaud partition or classification. The Couinaud partition is described in C. Couinaud, Le foie: 6tudes anatomiques et chirurgicales, Masson & Cie, 1957. This partition divides the liver into multiple functional segments. Three large hepatic veins that drain low-oxygen blood from the liver divide the liver into four venous segments. The portal vein, which supplies the liver with blood, further divides the liver into superior and inferior regions. The functional segments are typically wedge-shaped and the borders of the segments are planes or semi flat-curved surfaces, that can be referred to as intersegmental planes.

The segmentation can be performed by first segmenting the vascular structure and the parenchyma of the liver and then performing the Couinaud classification. Approaches of automatically segmenting the liver into these functional segments are, e.g., known from Le, D. C. et al., Functional Segmentation for Preoperative Liver Resection Based on Hepatic Vascular Networks, IEEE Access, vol. 9, pp. 15485-15498 (2021) and from Lebre M.A. et al., Automatic segmentation methods for liver and hepatic vessels from CT and MRI volumes, applied to the Couinaud scheme, Comput Biol Med.,

110:42-51 (2019).

Alternatively, the described method could use a manual or assisted segmentation of the tumor and/or the functional segments of the liver.

When the depicted liver comprises multiple tumors, separate resections can be planned for each of the tumors. The separate resections can preferably be performed during a single medical procedure. It is however also possible, that a single resection can be used to remove a part of the liver that comprises all or at least multiple ones of the tumors. The removal of a part of the liver comprising multiple tumors can especially be advantageous, when the multiple tumors are located within or at least partially within the same functional segment of the liver and are closely spaced. It is, e.g., possible to treat the part of the liver or of the respective functional segment comprising the multiple tumors as the segmented tumor, e.g., by first segmenting the individual tumors and then treating the convex hull including all of these tumors as the segmented tumor for the further steps of the described method.

In the case, that the partial removal of the respective selected functional segment is selected, at least one cutting parameter that parametrizes a given equation to describe a cutting surface for surgically cutting out the tumor can additionally be determined based on the segmented tumor, wherein either the cutting parameter and/or the cutting surface can be provided as part of the proposed liver resection plan or wherein the cutting parameter can be modified by an input acquired from a user and the resulting modified cutting parameter and/or a modified cutting surface described by parametrizing the given equation using the modified cutting parameter can be provided as part of the proposed liver resection plan.

The method can therefore not only determine, when it is advantageous to only partially remove a given functional segment comprising the tumor. It can further aid a clinician or any other user by suggesting a concrete cutting surface.

The determination of the cutting surface can also be used to evaluate the risk and/or the severity of the proposed intervention, e.g., during an initial evaluation of different treatment options.

The proposed cutting surface can optionally be further modified by a user during the planning, e.g., to adjust the cutting surface to the skill level and/or preferences of a clinician.

A graphical representation of the cutting surface, preferably a superposition of a representation of the medical image dataset and a representation of the cutting surface, can be generated and output to the user prior to the acquisition of the input from the user. Alternatively or additionally, a graphical representation of the modified cutting surface, preferably a superposition of a representation of the medical image dataset and a representation of the modified cutting surface, can be generated and output to the user.

It is especially possible that a further user input is acquired after visualizing the modified cutting surface to allow for a further modification of the cutting parameter and therefore the cutting surface while having graphic feedback concerning the changes. Visualizing the original and/or modified cutting surface allows the user to modify the cutting parameter and therefore the cutting surface in a similar way to the work flow of known tools for planning liver resections while still providing a promising starting point, namely the proposed cutting surface.

The selection, whether a complete removal or a partial removal of the respective selected functional segment is to be performed according to the proposed liver resection plan, can depend, either in all cases or only in cases in which a trigger condition depending on the segmented tumor is fulfilled, on the distance between the or a cutting surface, for surgically cutting out the tumor, from a vein that is segmented in the medical image dataset and/or from a further one of the functional segments that is not selected for complete removal, wherein the cutting surface is determined by parametrizing the or a given equation by the or a cutting parameter based on the segmented tumor.

The selection, whether complete or partial removal is suggested, can therefore take the actual shape of the proposed cutting surface into account. It is, e.g., possible, that the tumor would be sufficiently far away from veins and other relevant structures, but that limitations concerning achievable cutting surfaces would lead to the necessity of a cut quite close to a vein or another functional segment. In these cases, a complete removal of functional segment can be advantageous to reduce the risks caused by the resection.

The trigger condition can especially be always fulfilled, when a complete removal of the respective selected functional segment is not already indicated due to other reasons that are discussed below. The decision, whether to completely or partially remove the respective selected functional segment, can therefore be a two-step process. In the first step, one or multiple reasons for the removal of the complete segment, e.g., the size of the tumor exceeding a threshold, can be checked and if the partial removal of the respective selected functional segment seems advantageous after this first step, the cutting surface can be determined and considered in the next step as discussed above.

The cutting parameter can be determined by performing the additional steps of:

- determining a tumor region of the medical image dataset comprising the tumor or the part of the tumor extending into the respective selected functional segment based on the segmentation of the tumor,
- selecting a surface point on a chosen section of a surface of the respective selected functional segment, wherein the surface of the respective selected functional segment is given by the segmentation of the respective selected functional segment, wherein the chosen section of the surface either forms an outer surface of the liver or faces towards a further one of the selected functional segments for which a complete removal is selected in the proposed liver resection plan, and

- determining the cutting parameter in such a way, that the cutting surface separates a resected part of the respective selected functional segment, which comprises the tumor region and the selected surface point and which is to be removed according to the proposed liver resection plan, from a remaining part of the respective selected functional segment, which is to remain according to the proposed liver resection plan.

Due to the discussed selection of the surface point, the surface point is part of a surface section that is either directly accessible during that resection procedure or accessible after the removal of the further selected functional segment. Therefore, the inclusion of the surface point and the tumor section within the resected part ensures that the cutting surface is actually accessible during the resection, especially when the parametrization of the cutting surface is limited in such a way that the cutting surface intersects with the section of the surface comprising the surface point.

The tumor region can be determined by determining the vertices of the convex hull for the segmented tumor and by shifting these vertices away from the centroid of this convex hull by a given safety margin to provide tumor region vertices. By including such a tumor region within the resected part, the minimum distance between the cutting surface and the tumor cannot drop below the safety margin. Therefore, minor variations in the cutting surface actually achieved during the resection, e.g., due to a limited accuracy of clinician performing the cut, do not damage the tumor or lead to an incomplete removal of the tumor.

The convex hull of a segment is defined as the smallest convex region that contains the segment. Medical image datasets are often voxel based. Coordinates of vertices can be quantized to voxel positions or fractional voxel positions, e.g., floating-point or fixed-point coordinates, can be used during processing.

When multiple tumors are to be removed in the same resection, the convex hull for all of these tumors can be used instead of the convex hull for single tumor when determining the tumor region.

The safety margin, can, e.g., be chosen to be between 1 mm and 5 mm. The used safety margin can be a user adjustable parameter, e.g., to adjust the safety margin depending on the known skill level of the clinician, or it can be fixed or, e.g., depend on properties of the tumor.

The cutting parameters can be determined by

- determining further vertices that are vertices of the convex hull comprising the tumor region and the selected surface point,

- selecting those of the further vertices that are also tumor region vertices, and
- fitting the cutting surface to the selected further vertices under the condition that none of the selected further vertices are located in the remaining part of the respective selected functional segment.

The determination of the further vertices and the selection of the selected further vertices as discussed above ensures that those tumor region vertices that close of the tumor region in the direction of the section of the surface comprising the surface point are not taken into account during the fitting of the cutting surface. Especially when the given equation is chosen in such a way that the complexity of the shape of the cutting surface is limited, e.g., by only allowing the generation of cutting surfaces without points of inflection, the discussed fitting of the cutting surface leads to a cutting surface that can actually be cut along by a clinician without requiring unusual skill.

The complexity of the required cut depends on the given equation. Preferably the equation can describe a quadratic surface, especially a parabolic shape. In this case the presence of points of inflection in the cutting surface can be robustly avoided. Approaches for fitting various quadratic surfaces to a group of points are well known and are, e.g., discussed in Andrews J., Séquin C.H., Type-Constrained Direct Fitting of Quadric Surfaces, Computer-Aided Design and Applications, 11:1, 107-119 (2014).

It can be possible to limit the range of the cutting parameter or at least one of the cutting parameters to ensure that a cut can actually be performed by a clinician. The limitation can, e.g., also depend on a known skill level of the clinician or can be selectable by a user. It is also possible that the given equation can be chosen to, e.g., accommodate clinicians with different preferences and/or skill levels. By limiting the surface, e.g., to an elliptic cone, the complexity of the cut can be further reduced. Alternatively higher order surfaces could be allowed for experienced clinicians.

Additionally or alternatively to a selection of the given equation and/or to a limitation of the available range of the respective cutting parameter, a skill level of a clinician can, e.g., be taken into account by varying the safety margin.

The selected surface point can be selected in such a way, that the distance between the selected surface point and the tumor region is minimized. This helps to minimize the volume of healthy liver tissue that is removed during the resection.

The given equation can define the cutting surface to be a paraboloid. In this case the cutting surface can be open towards the surface section comprising the surface point and widen towards this surface. This allows for a relatively easy cutting along the cutting surface while keeping the amount of removed healthy tissue small.

Fitting can be performed in this case by, e.g.,

first defining a vector from the centroid of the tumor region to the selected surface point and then rotating the vector and the selected further vertices in such a way, that the vector is aligned with the z-axis. The paraboloid can then be described using the equation

$$z = \frac{(x\text{-}x_0)^2}{a} + \frac{(y\text{-}y_0)^2}{b},$$

wherein a and b are cutting parameters giving the width of the paraboloid in the x- and y-direction and the cutting parameters $x_0$ and $y_0$ define the position of the apex of the paraboloid in the rotated coordinate system. The angles of rotation of the discussed rotation of the vector and the vertices can be further cutting parameters, thereby allowing the cutting surface to be realigned with the original coordinate system of the medical image dataset. Alternatively, the cutting parameters could, e.g., directly parametrize the fitted paraboloid in the original coordinate system.

The or a surface of the respective selected functional segment can be given by the segmentation of the respective selected functional segment. A minimum distance between the segmented tumor and a section of the surface that either forms the or an outer surface of the liver or that is adjacent to a further selected segment, for which a complete removal is selected, can be determined. The complete removal of the respective selected functional segment can then, e.g., be selected when the determined minimum distance reaches or exceeds a respective threshold. In other words, the partial removal of the respective selected functional segment can optionally only be chosen when the segmented tumor is sufficiently close to a surface section that is accessible during the resection. For tumors deep within a functional segment, a complete removal of that functional segments is often advantageous.

The complete removal of the respective selected functional segment can be selected when the volume and/or the maximum diameter of the segmented tumor reaches or exceeds a respective threshold and/or when the distance of the segmented tumor to the or a vein that is segmented in the medical image dataset reaches or falls below a respective threshold. The threshold for the distance of the segmented tumor to a vein can, e.g., be twice the safety margin discussed above and/or between 2 and 10 mm.

At least one measure for the risk and/or the severity of the intervention can be determined for the proposed liver resection plan and be provided as part of the proposed liver resection plan. This measure can, e.g., be or depend on the removed and/or remaining liver volume and/or the minimum distance of the cutting surface to a vein. The determined measure can especially be used for comparing a resection according to the proposed liver resection plan to other treatment options, e.g., to a treatment of the tumor by ablation or by radioembolization. Since the proposed liver resection plan can be generated automatically, the risk and/or severity of the intervention can be quickly determined with good accuracy. The risk and/or severity analysis and/or a comparison to other methods can therefore be performed on the fly, e.g., during a cancer board without any elaborate preparation.

Due to the ability to quickly modify the resection plan, especially the cutting surface, by the interactive method discussed above, preferences and skill levels of the clinician that will actually be performing the intervention, can also be taken into account. Additionally or alternatively, preferences and skill levels of the clinician can be taken into account by limiting the cutting parameters, by choosing a specific given equation, etc. Therefore, the discussed method even allows to take the preference and skill levels of available clinicians into account, when judging the risk and/or severity of the intervention and/or when comparing it to other treatment methods.

Besides the inventive method the invention also concerns a data processing system that is designed to carry out the computer implemented method according to the present invention. The data processing system can, e.g., be a desktop computer, a server or be implemented as a cloud solution. Additionally or attentively, it can be integrated into a medical imaging device.

The data processing system can have hardware and/or software interfaces for receiving the medical image data set and/or for providing the proposed liver resection plan. It can comprise or control a display device for visualizing the cutting surface and/or input means for receiving a user input as discussed above with respect to the method according to the present invention.

The invention also concerns a computer program that comprises instructions to carry out the method according to the present invention.

Additionally, the invention concerns a computer-readable medium comprising the computer program according to the present invention.

Any features discussed with respect to the method according to the present invention can be transferred to the data processing system, the computer program, and the computer-readable medium with the advantages given above and vice versa.

Other objects and features of the present invention will become apparent from the following detailed description considered in conjunction with the accompanying drawings. The drawings, however, are only principle sketches designed solely for the purpose of illustration and do not limit the invention. The drawings show:

Fig. 1    a flowchart of an exemplary embodiment of the computer implemented method for planning a liver resection according to the present invention,

Fig. 2    an exemplary embodiment of a data processing system according to the present invention and its interaction with further relevant components,

Fig. 3      a liver comprising a tumor and the segmentation of the liver into functional segments,

Fig. 4      an illustration of the determination of a cutting surface in an exemplary embodiment of the computer implemented method for planning a liver resection according to the present invention.

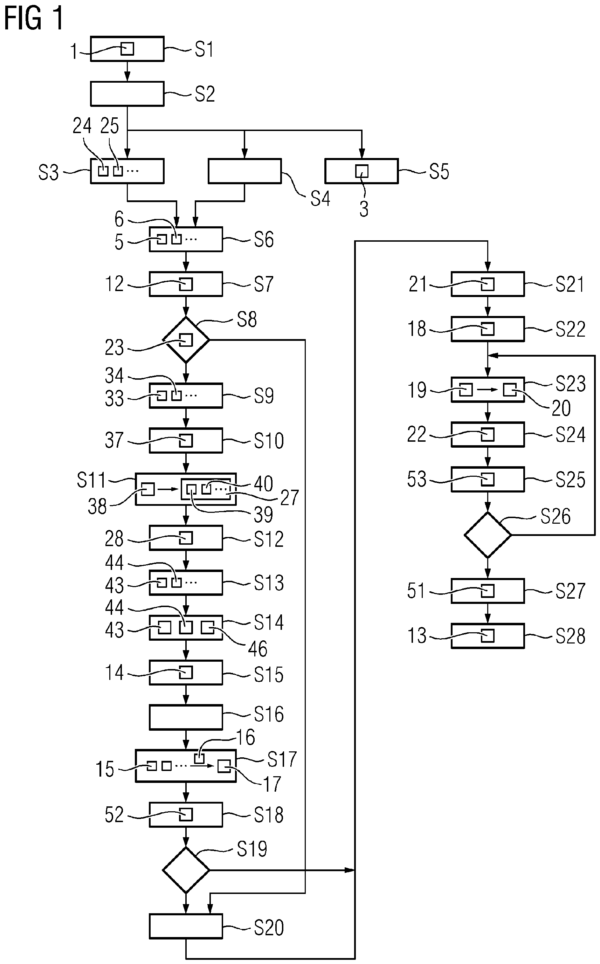

Fig. 1 shows a flowchart of a computer implemented method for planning a liver resection. the flowchart shows a detailed example comprising many optional steps to illustrate possible implementations of certain functionalities and optional additional features. Therefore, an overview of the most relevant features and steps will be given before discussing the individual steps shown in fig. 1.

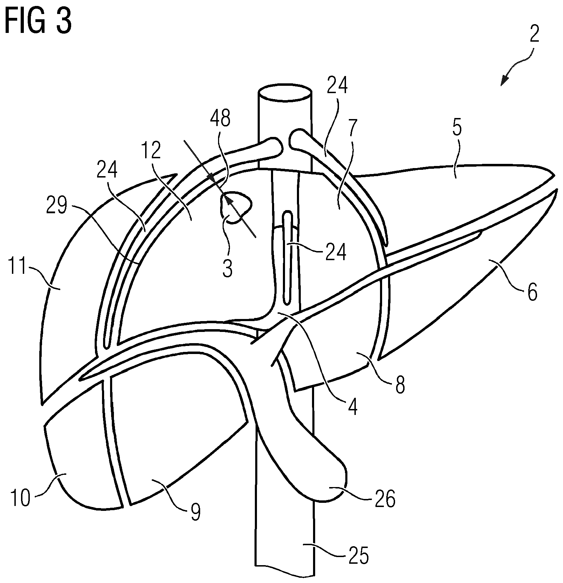

After receiving a three-dimensional medical image dataset 1 in step S1, the medical image dataset 1 is processed over multiple steps to determine and provide the proposed liver resection plan 13 in step S28. The medical image dataset 1 comprising a depiction of a liver 2, comprising a tumor 3. A schematic drawing of such a liver 2 is shown in fig. 3.

In the step S3, S4 and S6 multiple functional segments 4-12 of the liver 2 are segmented and in step S5 the tumor 3 is segmented in the medical image dataset 1. In step S7 any functional segments 12 that comprises a tumor 3 or into which the tumor 3 extends are selected. Since only the functional segment 12 comprises a tumor in the example, only this functional segment 12 is selected.

In the course of the steps S8 to S20, the selected functional segment 12 and the tumor 3 are analyzed to decide, whether a complete removal or a partial removal of the selected functional segment 12 is to be performed in the proposed liver resection plan 13.

The steps S21 to S27 are optional steps that allow a user to modify the liver resection plan 13 and to support the user in judging the risk and/or the severity of the planned intervention.

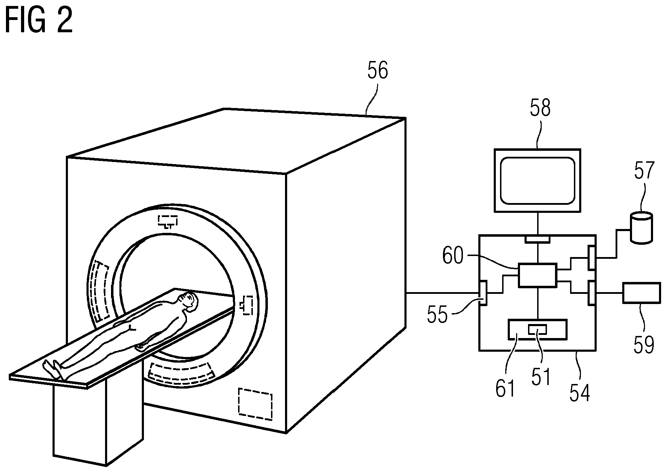

Two options for receiving the three-dimensional medical image dataset 1 in step S1 will now be discussed with additional reference to fig. 2 that shows an example for a data processing system 54 that is designed to implement the discussed method. The data processing system 54 can, e.g., be a desktop computer or some other programmable computing device. In the example a processor 60 executes a computer program 51 stored in the memory 61 to implement the various steps shown in fig. 1.

Data can be received and sent via various interfaces 55 of the data processing system 54. Concerning the reception of the three-dimensional medical image dataset 1, two potential sources are shown in fig. 2. In the simplest case the medical image dataset 1 can be directly received from a medical imaging device 56. In the example, a CT-scanner is shown. In other embodiments the medical imaging device 56 could however, e.g., be a MR-scanner or a molecular imaging device. It is however also possible to receive a previously recorded medical image dataset 1 from a database 57.

In step S2 the liver is segmented in the medical image dataset 1. Preferably an automatic segmentation is performed. an automatic segmentation of a liver 2 is well known in the prior art and will not be discussed in detail.

In step S3 the various veins 24-26 in the area of the liver 2 are segmented. In step S4 the parenchyma or tissue of the liver is segmented and in step S5 the tumor 3 is segmented. Preferably these segmentations are performed automatically. Approaches for segmenting areas comprising different tissues and blood vessels are well known in the prior art.

In step S6 of the segmentation of the functional segments 4 to 12 shown in fig. 3 is performed based on the results of steps S3 and S4. As already discussed in the general part of the description, approaches for an automatic segmentation such functional segments 4-12 are known. It is, e.g., possible to classify the individual voxels of the parenchyma segmented in step S4 using their respective position with respect to the various veins 24 to 26 segmented in step S3.

In step S7 the functional segments 12 comprising a tumor 3 are selected. In the example only a single functional segmented 12 comprises the tumor 3. If the tumor 3 would extent through multiple functional segments 4-12 and/or if multiple ones of the functional segments 4-12 would comprise a respective tumor 3, all of these segments would be selected.

When multiple segments are selected, it is possible, that only some of the selected functional segments are completely removed and some are only partially removed in the proposed liver resection plan 13. This can be relevant for determining the cutting surface 17 that is used in partial resections as already discussed in the general part of the description.

In step S8 trigger condition 23 is evaluated for the selected functional segmented 12. A fulfillment of the trigger condition will lead to a more detailed analysis, if a partial removal of the selected functional segmented 12 is advantageous in the steps S9 to S19 as discussed in more detail below. If the trigger condition 23 is not fulfilled, the steps S9 to S19 are skipped and the complete removal of the selected functional segmented 12 is selected in step S 20.

The trigger condition 23 can, e.g., only be fulfilled when the diameter 49 and/or volume of the segmented tumor 3 is below a respective given threshold value and/or when the minimum distance 50 between the tumor 3 and any of the segmented veins 24 to 26 exceeds a given threshold value.

Additionally, the fulfillment of the trigger condition 23 can require that the distance 48 between the tumor 3 and a section of the surface 29 of the selected functional segmented 12 that either forms and outer surface 30 of the liver 2 or that is adjacent to a further selected functional segment, which is to be completely removed, is smaller than a threshold value.

In other words, the trigger condition 23 leads to the selection of a complete removal of the selected functional segmented 12 if a partial removal would be problematic even before taking a cutting surface 17 for cutting out the resected part 31 into account.

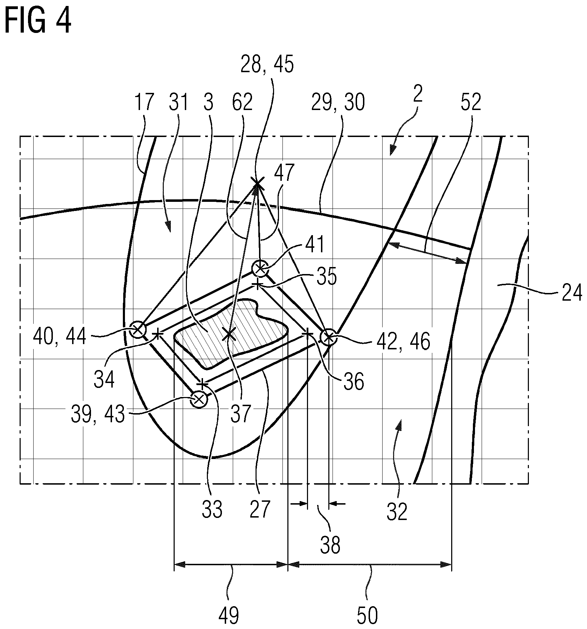

An example of the determination of a cutting surface 17 for a partial removal of the selected functional segment 12 is shown schematically in fig. 4 and will now be discussed. Fig. 4 shows a section of an exemplary medical image dataset 1 that is processed by the method. For simplicity's sake only a two-dimensional plane of the three-dimensional medical image dataset 1 is shown. The underlying grid shown in fig. 4 represents individual voxels and therefore the limits of the resolution of the medical image data set 1. For reasons of clarity, a rather low resolution is shown in fig. 4. Real implementations can use notably higher resolutions.

The shown area of the medical image data 1 shows the area of the liver 2 comprising the tumor 3. For reasons of clarity, the area of the liver 2 and the functional segment 12 comprising the tumor 3 is not shaded and only the surface 29 of the functional segmented 12 that also forms the outer surface 30 of the liver 2 his shown. The area below the surface 29 in fig. 4 is part of the functional segmented 12 and the area above lies outside of the liver 2.

In step S9, vertices 33-36 of the convex hull for the segmented tumor 3 are determined. In the example the convex hull and therefore the position of the vertices 33-36 is determined at the resolution given by the voxel grid shown in fig. 4.

In step S10 the centroid 37 for this convex hull is determined and in step S11 the vertices 33-36 are shifted away from the centroid 37 by a given safety margin 38 to provide tumor region vertices 39-42 of a determined tumor region 27. This step and the further processing steps can be performed at a sub-voxel resolution to allow for a higher accuracy.

In step S12 a surface point 28 on the section of the surface 29 of the functional segment 12 that forms an outer surface of the liver is selected in such a way, that the distance 47 between the selected surface point 28 and the tumor region 27 is minimized. In datasets for which a different one of the functional segments 4-12 would be completely removed, the surface point 28 could also be placed on a section of the surface 29 that is adjacent to the completely removed functional segment.

In step S 13, further vertices 43-46 are determined that are vertices of the convex hull comprising the tumor region 27 and the selected surface point 28.

In step S14 those of the further vertices 43-46 that are also tumor region vertices 39-42 are selected and the cutting surface 17 is fitted to the selected further vertices 43, 44, 46 in the steps S15 to S17 under the condition that none of the selected further vertices 43, 44, 46 are located in the remaining part 32 of the selected functional segment 12 that lies below the cutting surface 17 and in the example in fig. 4.

In the example, the cutting surface 17 is a paraboloid that is described by a given equation 16 parametrized by multiple cutting parameters 14, 15. As already discussed in the general part of the description, the cutting parameters 14, 15 can especially describe the orientation of the paraboloid with respect to the medical image dataset 1 and the position of the apex of the paraboloid and parameters describing the opening of the paraboloid in two orthogonal directions.

In the given example, the orientation of the paraboloid is determined in step S15 as cutting parameter 14. For this purpose, the vector 62 that extends from the centroid 37 to the selected surface point 28 is determined and the cutting parameter 14 is selected in such a way that it describes a rotation of the coordinate system in such a way that the vector 62 extends in the z-direction.

This rotation is then applied in step S16 to all of the selected further vertices 43, 44, 46. In step S17 the remaining cutting parameters 15 are then determined as the free parameters $a$, $b$, $x_0$ and $y_0$ in the equation describing the paraboloid

$$z = \frac{(x\text{-}x_0)^2}{a} + \frac{(y\text{-}y_0)^2}{b}.$$

This determination can, e.g., be performed using a least square fitting or other known fitting approaches under the additional condition, that the selected further vertices 43, 44, 46 are located within the resected part 31 of the selected functional segment 12.

As already described in the general part of the description, such an optimization of the cutting surface 17 allows for a minimization of the resected part 31 while still leading to a cutting surface 17 that can be followed by clinicians without requiring an unusual skill level. As already discussed in the general part of the description, the given equation 16 and/or limitations of the cutting parameters 14,15 could be chosen to adjust for various skill levels of the clinician.

In the step S18 the minimum distance 52 of the determined cutting surface 17 to the closest segmented vein 24 is determined, as schematically shown in fig. 4.

In step S 19 this distance 52 is then compared to a threshold. When the distance 52 falls below this threshold, a complete removal of the selected segmented 12 is chosen in step S20. When the cutting surface 17 approaches to closely to a vein, a partial resection would risk damaging the vein and it can therefore be advantageous to suggest a complete removal of the selected segmented 12 instead.

While it would be possible to immediately provide the proposed liver resection plan 13 after the steps S19 and S20, the example given in fig. 1 comprises additional steps that allow a user to interactively adjust the proposed liver resection plan 13 to his or her preferences and/or skill level. In step S21 a graphical representation 21 of the cutting surface 17, preferably a superposition of a representation of the medical image dataset 1 and the representation 21 of the cutting surface 17, is generated and output to the user via the display device 58 shown in fig. 2.

In step S 22 the user can then provide an input 18 to modify the cutting surface 17, e.g., by entering a modified cutting parameter 19 for one or multiple ones of the previously determined cutting parameters 14, 15, via the input device 59. The modified cutting parameter 19 is then used in step S23 to determine a modified cutting surface 20 and a graphical representation 22 of this modified cutting surface 20 that is output via the display device 58 in step S 24.

In step S 25 a further user input 53 can be acquired. If the further user input 53 indicates, that a further modification of the cutting surface 17 or in general of the proposed liver resection plan 13 is desired, step S 23 and the following steps can be repeated.

Otherwise, a measure for the risk and/or the severity of the intervention can optionally be determined for the proposed liver resection plan 13 in Step S27. Such a measure can be useful for comparing the proposed liver resection plan 13 to other treatment options, as already discussed in the general description. The measure can, e.g., be based on the volume of the resected part 31 and/or the remaining part 32 and/or on the distance 52 of the proposed cutting surface 17 to the closest vein 24, 25, 26.

The proposed liver resection plan 13 can then be provided in step S28.

Although the present invention has been described in detail with reference to the preferred embodiment, the present invention is not limited by the disclosed examples from which the skilled person is able to derive other variations without departing from the scope of the invention.

Independent of the grammatical term usage, individuals with male, female or other gender identities are included within the term.

**Claims**

1. Computer implemented method for planning a liver resection, comprising the steps of

   - receiving a three-dimensional medical image dataset (1) comprising a depiction of a liver (2) of a patient, the depicted liver (2) comprising a tumor (3),
   - segmenting multiple functional segments (4-12) of the liver (2) in the medical image dataset (1),
   - segmenting of the tumor (3) in the medical image dataset (1),
   - selecting at least one of the functional segments (4-12) that comprises the tumor (3) or into which the tumor (3) extends, based on the segmentation of the tumor (3) and the segmentation of the functional segments (4-12),
   - selecting for the respective selected functional segment (12), whether a complete removal or a partial removal of this selected functional segment (12) is to be performed in a proposed liver resection plan (13), wherein the selection is based on the segmented tumor (3), and
   - providing the proposed liver resection plan (13).

2. Computer implemented method according to claim 1, **characterized in that** in the case that the partial removal of the respective selected functional segment (12) is selected, at least one cutting parameter (14, 15) that parametrizes a given equation (16) to describe a cutting surface (17) for surgically cutting out the tumor (3) is additionally determined based on the segmented tumor (3), wherein either the cutting parameter (14, 15) and/or the cutting surface (17) are provided as part of the proposed liver resection plan (13) or wherein the cutting parameter (14, 15) is modified by an input (18) acquired from a user and the resulting modified cutting parameter (19) and/or a modified cutting surface (20) described by parametrizing the given equation (16) using the modified cutting parameter (19) are provided as part of the proposed liver resection plan (13).

3. Computer implemented method according to claim 2, **characterized in that** a graphical representation (21) of the cutting surface (17), preferably a superposition of a representation of the medical image dataset (1) and a representation (21) of the cutting surface (17), is generated and output to the user prior to the acquisition of the input (18) from the user and/or that a graphical representation (22) of the modified cutting surface (20), preferably a superposition of a representation of the medical image dataset (1) and a representation (22) of the modified cutting surface (20), is generated and output to the user.

4. Method according to one of the preceding claims, **characterized in that** the selection, whether a complete removal or a partial removal of the respective selected functional segment (12) is to be performed according to the proposed liver resection plan (13), depends, either in all cases or only in cases in which a trigger condition (23) depending on the segmented tumor (3) is fulfilled, on the distance (52) of the or a cutting surface (17), for surgically cutting out the tumor (3), from a vein (24) that is segmented in the medical image dataset (1) and/or from a further one of the functional segments (4-12) that is not selected for complete removal, wherein the cutting surface (17) is determined by parametrizing the or a given equation (16) by the or a cutting parameter (14, 15) based on the segmented tumor (3).

5. Computer implemented method according to one of the claims 2 to 4, **characterized in that** the cutting parameter (14, 15) is determined by performing the additional steps of:

- determining a tumor region (27) of the medical image dataset (1) comprising the tumor (3) or the part of the tumor (3) extending into the respective selected functional segment (12) based on the segmentation of the tumor (3),
- selecting a surface point (28) on a chosen section of a surface (29) of the respective selected functional segment(12), wherein the surface (28) of the respective selected functional segment is given by the segmentation of the respective selected functional segment (12), wherein the chosen section of the surface (12) either forms an outer surface (30) of the liver or faces towards a further one of the selected functional segments (12) for which a complete removal is selected in the proposed liver resection plan (13), and
- determining the cutting parameter (14, 15) in such a way, that the cutting surface (17) separates a resected part (31) of the respective selected functional segment (12), which comprises the tumor region (27) and the selected surface point (28) and which is to be removed according to the proposed liver resection plan (13), from a remaining part (32) of the respective selected functional segment (12), which is to remain according to the proposed liver resection plan (13).

6. Method according to claim 5, **characterized in that** the tumor region (27) is determined by determining the vertices (33-36) of the convex hull for the segmented tumor (3) and by shifting these vertices (33-36) away from the centroid (37) of this convex hull by a given safety margin (38) to provide tumor region vertices (39-42).

7. Method according to claim 6, **characterized in that** the cutting parameters are determined by

- determining further vertices (43-46) that are vertices of the convex hull comprising the tumor region (27) and the selected surface point (28),
- selecting those of the further vertices (43, 46) that are also tumor region vertices (39-42), and
- fitting the cutting surface (17) to the selected further vertices (43, 44, 46) under the condition that none of the selected further vertices (43, 44, 46) are located in the remaining part (32) of the respective selected functional segment (12).

8. Method according to one of the claims 5 to 7, **characterized in that** the selected surface point (28) is selected in such a way, that the distance (47) between the selected surface point (28) and the tumor region (27) is minimized.

9. Method according to one of the preceding claims, **characterized in that** the given equation (16) defines the cutting surface (17) to be a paraboloid.

10. Computer implemented method according to one of the preceding claims, **characterized in that** the or a surface (29) of the respective selected functional segment (12) is given by the segmentation of the respective selected functional segment (12), wherein a minimum distance (48) between the segmented tumor (3) and a section of the surface (29) that either forms the or an outer surface (30) of the liver (2) or that is adjacent to a further selected segment (12), for which a complete removal is selected, is determined, wherein the complete removal of the respective selected functional segment (12) is selected when the determined minimum distance (48) reaches or exceeds a respective threshold.

11. Computer implemented method according to one of the preceding claims, **characterized in that** the complete removal of the respective selected functional segment (12) is selected when the volume and/or the maximum diameter (49) of the segmented tumor (3) reaches or exceeds a respective threshold and/or when the distance (50) of the segmented tumor (3) to the or a vein (24) that is segmented in the medical image dataset (1) reaches or falls below a respective threshold.

12. Computer implemented method according to one of the preceding claims, **characterized in that** the at least one measure (51) for the risk and/or the severity of the intervention is determined for the proposed liver resection plan (13) and provided as part of the proposed liver resection plan (13).

13. Data processing system, **characterized in that** it is designed to carry out the computer implemented method according to one of the preceding claims.

14. Computer program, **characterized in that** it comprises instructions to carry out the method according to one of the claims 1 to 12.

15. Computer-readable medium comprising the computer program (51) according to claim 14.

# FIG 1

# FIG 2

# FIG 3

FIG 4

Europäisches
Patentamt
European
Patent Office
Office européen
des brevets

# EUROPEAN SEARCH REPORT

Application Number

EP 23 17 1634

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2015/063668 A1 (YOU HEECHEON [KR] ET AL) 5 March 2015 (2015-03-05)<br>* paragraph [0038] - paragraph [0045]; figure 1 *<br>* paragraph [0081] - paragraph [0085] * | 1-3,<br>13-15<br>4-12 | INV.<br>A61B34/10<br>G06T7/10 |
| X<br>A | US 2013/144160 A1 (SAKURAGI FUTOSHI [JP]) 6 June 2013 (2013-06-06)<br>* paragraph [0041] - paragraph [0059]; figures 1-4 * | 1-5,8,<br>13-15<br>6,7,9-12 | |
| X<br>A | US 2014/333617 A1 (MIYAMOTO MASAKI [JP]) 13 November 2014 (2014-11-13)<br>* paragraph [0085] - paragraph [0097]; figures 4A-5B * | 1-3,<br>13-15<br>4-12 | |

TECHNICAL FIELDS
SEARCHED      (IPC)

A61B

The present search report has been drawn up for all claims

| Place of search | Date of completion of the search | Examiner |
|---|---|---|
| Munich | 19 September 2023 | Geiss, Oana |

## ANNEX TO THE EUROPEAN SEARCH REPORT
## ON EUROPEAN PATENT APPLICATION NO.

EP 23 17 1634

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2023

| Patent document cited in search report | | Publication date | Patent family member(s) | | Publication date |
|---|---|---|---|---|---|
| US 2015063668 | A1 | 05-03-2015 | KR | 20130100758 A | 11-09-2013 |
| | | | US | 2015063668 A1 | 05-03-2015 |
| US 2013144160 | A1 | 06-06-2013 | CN | 103068313 A | 24-04-2013 |
| | | | EP | 2604189 A1 | 19-06-2013 |
| | | | JP | 5011426 B2 | 29-08-2012 |
| | | | JP | 2012034988 A | 23-02-2012 |
| | | | US | 2013144160 A1 | 06-06-2013 |
| | | | WO | 2012020547 A1 | 16-02-2012 |
| US 2014333617 | A1 | 13-11-2014 | JP | 5785214 B2 | 24-09-2015 |
| | | | JP | 2014217549 A | 20-11-2014 |
| | | | US | 2014333617 A1 | 13-11-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MISE, Y. et al.** Virtual liver resection: computer-assisted operation planning using a three-dimensional liver representation. *J Hepatobiliary Pancreat Sci.,* 2013, vol. 20, 157-164
- **CHRIST B. et al.** Computational Modeling in Liver Surgery. *Front Physiol.,* 2017, vol. 8
- Functional Segmentation for Preoperative Liver Resection Based on Hepatic Vascular Networks. **LE, D. C. et al.** IEEE Access. 2021, vol. 9, 15485-15498

- **LEBRE M.A. et al.** Automatic segmentation methods for liver and hepatic vessels from CT and MRI volumes, applied to the Couinaud scheme. *Comput Biol Med.,* 2019, vol. 110, 42-51
- **ANDREWS J. ; SÉQUIN C.H.** Type-Constrained Direct Fitting of Quadric Surfaces. *Computer-Aided Design and Applications,* 2014, vol. 11 (1), 107-119